# EUROPEAN PATENT APPLICATION

(11) **EP 4 293 675 A1**
(43) Date of publication of application: **20.12.2023**
(21) Application number: 23178978.5
(22) Date of filing: 13.06.2023
(51) Int. Cl.: G16H 20/40, G16H 40/67, A61B 34/00, G06Q 10/06, G06Q 10/0631, G06Q 10/0633, G06Q 10/0639, G06Q 50/00, G16H 40/20, G16H 70/20, A61B 34/20, G06V 20/52, G09B 23/28, G16H 30/40

(54) **SYSTEMS AND METHODS FOR NON-COMPLIANCE DETECTION IN A SURGICAL ENVIRONMENT**

(30) Priority: 14.06.2022 US 202263366399 P
(71) Applicant: Stryker Corporation, Kalamazoo, MI 49002 (US)
(72) Inventor: TIWARY, Vijay Kumar, Kalamazoo, 49002 (US); HASTINGS, Sean Victor, Kalamazoo, 49002 (US); BHARDWAJ, Gaurav, Kalamazoo, 49002 (US)
(74) Representative: V.O.

(57) **Abstract**

The present disclosure relates generally to improving surgical safety, and more specifically to techniques for automated detection of non-compliance to surgical protocols in a surgical environment such as an operating room. An exemplary method comprises: receiving one or more images of the operating room captured by one or more cameras; detecting a surgical milestone associated with a surgery in the operating room using a first set of one or more trained machine-learning models based on the received one or more images; detecting one or more activities in the operating room using a second set of one or more trained machine-learning models based on the received one or more images; and determining, based on the detected one or more activities and a surgical protocol associated with the detected surgical milestone, that an instance of non-compliance to the surgical protocol has occurred in the operating room.

## Description

### CROSS-REFERENCE TO RELATED APPLICATIONS

This application claims the benefit of U.S. Provisional Application No. 63/366,399, filed June 14, 2022, the entire contents of which are hereby incorporated by reference herein.

### FIELD

The present disclosure relates generally to improving surgical safety, and more specifically to techniques for automated detection of non-compliance to surgical protocols in a surgical environment such as an operating room (OR).

### BACKGROUND

In healthcare institutions that conduct surgical procedures, it is common practice to engage independent consultants to audit surgical procedures when surgical site infections (SSI) trend upward to ascertain potential causes for the undesired trend. However, it is impractical for a consultant to observe all aspects of all surgeries occurring at an institution. Thus, the analysis and recommendations by consultants can be inaccurate, inefficient, and expensive. There is a lack of an automated system to improve the patient care and outcomes through a systemic review of care against the defined criteria of surgical protocols, procedures, and environments.

### SUMMARY

Disclosed herein are exemplary devices, apparatuses, systems, methods, and non-transitory storage media for determining non-compliance to surgical protocols in an operating room. Examples of the present disclosure can automate auditing of surgical workflows and improve the efficiency and accuracy of the audit logs. Some examples of the present disclosure include a system that employs a plurality of cameras in an operating room and processes live video streams using machine-learning algorithms such as object detection and tracking techniques to detect (e.g., in real time) instances of non-compliance when required protocols have been violated. In some examples, the machine-learning algorithms can be trained to monitor activities in the surgical workflow and recognize: adherence to operating room preparation and turnover protocols, non-compliance with sterile protocol, non-compliance with surgical attire, etc. The system can provide alerts in real time for certain protocol violations to prevent SSIs. Additionally, the system can provide suggestions for retraining opportunities and protocol enhancements. Accordingly, examples of the present disclosure provide an efficient and accurate mechanism for conducting surgical audits and improve surgical safety and patient outcomes in ongoing and future surgeries.

An exemplary method for determining non-compliance to surgical protocols in an operating room comprises: receiving one or more images of the operating room captured by one or more cameras; detecting a surgical milestone associated with a surgery in the operating room using a first set of one or more trained machine-learning models based on the received one or more images; detecting one or more activities in the operating room using a second set of one or more trained machine-learning models based on the received one or more images; and determining, based on the detected one or more activities and a surgical protocol associated with the detected surgical milestone, that an instance of non-compliance to the surgical protocol has occurred in the operating room.

Optionally, the method further comprises: determining a severity level of the instance of non-compliance to the surgical protocol.

Optionally, the method further comprises: determining that the severity level meets a predefined severity threshold; in accordance with the determination that the determined severity level meets the predefined severity threshold: generating an alert.

Optionally, the method further comprises: determining that the severity level does not meet the predefined severity threshold; in accordance with the determination that the severity level does not meet the predefined severity threshold: foregoing generating the alert.

Optionally, the alert is auditory, graphical, textual, haptic, or any combination thereof.

Optionally, the method further comprises: calculating an audit score for the surgery based on the instance of non-compliance to the surgical protocol.

Optionally, the surgical milestone is a first surgical milestone of the surgery and the surgical protocol associated with the surgical milestone is a first surgical protocol. The method further comprises: determining that an instance of non-compliance to a second surgical protocol associated with a second surgical milestone has occurred in the operating room; and calculating the audit score for the surgery based on the instance of non-compliance to the first surgical protocol and the instance of non-compliance to the second surgical protocol.

Optionally, the audit score is based on a weighted calculation of the instance of non-compliance to the first surgical protocol and the instance of non-compliance to the second surgical protocol.

Optionally, the method further comprises: comparing the audit score against a predefined audit score threshold.

Optionally, the predefined audit score threshold is associated with a type of the surgery in the operating room.

Optionally, the method further comprises: displaying the audit score on a display; and saving the audit score to an electronic medical record.

Optionally, the method further comprises: identifying a change to the surgical protocol; and outputting a recommendation based on the identified change to the surgical protocol.

Optionally, identifying a change to the surgical protocol comprises: identifying a correlation between an outcome of the surgery in the operating room and the instance of non-compliance to the surgical protocol.

Optionally, the method further comprises: recommending retraining of the surgical protocol based on the instance of non-compliance to the surgical protocol.

Optionally, the method further comprises: determining an identity or a surgical function of a person associated with the instance of non-compliance; and determining whether to recommend a change to the surgical protocol or to recommend retraining of the surgical protocol at least partially based on the identity or the surgical function of the person associated with the instance of non-compliance.

Optionally, the first set of one or more trained machine-learning models is the same as the second set of one or more trained machine-learning models.

Optionally, the first set of one or more trained machine-learning models is different from the second set of one or more trained machine-learning models.

Optionally, the one or more activities include: linen changing on a surgical table; cleaning of the surgical table; wiping of the surgical table; application of a disinfectant; introduction of a surgical equipment; preparation of the surgical equipment; entrance of a person into the operating room; exiting of the person out of the operating room; opening of a door in the operating room; closing of the door in the operating room; donning of surgical attire; contamination of sterile instruments; contact between anything sterile and a non-sterile surface; preparation of a patient; usage of one or more blood units; usage of one or more surgical sponges; usage of one or more surgical swabs; collection and/or disposal of waste; fumigation; sterile zone violation; a conducted time-out; a conducted debriefing; fogging; or any combination thereof.

Optionally, the second set of one or more trained machine-learning models is configured to detect and/or track one or more objects in the operating room.

Optionally, the one or more objects include: one or more surgical tables; one or more surgical lights; one or more cleaning supplies; one or more disinfectants; one or more linens; one or more surgical equipment; one or more patients; one or more medical staff members; attire of the one or more medical staff members; one or more doors in the operating room; one or more blood units; one or more surgical sponges; one or more surgical swabs; or any combination thereof.

Optionally, the attire of the one or more medical staff members includes: a surgical mask, a surgical cap, a surgical glove, a surgical gown, or any combination thereof.

Optionally, the one or more surgical equipment includes: one or more imaging devices, one or more monitoring devices, one or more surgical tools, or any combination thereof.

Optionally, the method further comprises: calculating a ratio between medical staff members and patients in the operating room.

Optionally, detecting the surgical milestone comprises: obtaining, from the first set of one or more trained machine-learning models, one or more detected objects or events; and determining, based upon the one or more detected objects or events, the surgical milestone.

Optionally, the method comprises determining that the severity level meets a predefined severity threshold, and in accordance with the determination that the determined severity level meets the predefined severity threshold starting an intervention. The method may further comprise: determining that the severity level does not meet the predefined severity threshold, and in accordance with the determination that the severity level does not meet the predefined severity threshold foregoing starting the intervention.

Optionally, the intervention is configured for drawing attention to meeting of the severity level. Optionally, the system may alert or notify the OR team of the severe protocol infraction. The alert may be visual, auditory, haptic, or any combination thereof. The alert may comprise an indication on one or more OR displays and/or one or more OR dashboards. The alert may optionally comprise a video or a textual description of the detected infraction and/or how severe it is. Optionally, the intervention is configured for at least momentarily pausing an operation. Optionally, the intervention comprises one or more of: altering the OR lighting, such as dimming the lights or setting the lights to higher brightness; modifying a view on a monitor, such as blocking or blurring the view, e.g. a camera view; blocking or providing haptic feedback on controls, e.g. of a surgical robot; blocking or providing, e.g. haptic, feedback on medical equipment, such as a diagnostic imaging device, an anesthesia machine, a staple gun, a retractor, a clamp, an endoscope, an electrocautery tool; or the like.

An exemplary system for determining non-compliance to surgical protocols in an operating room comprises: one or more processors; a memory; and one or more programs, wherein the one or more programs are stored in the memory and configured to be executed by the one or more processors, the one or more programs including instructions for: receiving one or more images of the operating room captured by one or more cameras; detecting a surgical milestone associated with a surgery in the operating room using a first set of one or more trained machine-learning models based on the received one or more images; detecting one or more activities in the operating room using a second set of one or more trained machine-learning models based on the received one or more images; and determining, based on the detected one or more activities and a surgical protocol associated with the detected surgical milestone, that an instance of non-compliance to the surgical protocol has occurred in the operating room.

Optionally, the one or more programs further include instructions for: determining a severity level of the instance of non-compliance to the surgical protocol.

Optionally, the one or more programs further include instructions for: determining that the severity level meets a predefined severity threshold; in accordance with the determination that the determined severity level meets the predefined severity threshold: generating an alert.

Optionally, the one or more programs further include instructions for: determining that the severity level does not meet the predefined severity threshold; in accordance with the determination that the severity level does not meet the predefined severity threshold: foregoing generating the alert.

Optionally, the alert is auditory, graphical, textual, haptic, or any combination thereof.

Optionally, the one or more programs further include instructions for: calculating an audit score for the surgery based on the instance of non-compliance to the surgical protocol.

Optionally, the surgical milestone is a first surgical milestone of the surgery and the surgical protocol associated with the surgical milestone is a first surgical protocol. The one or more programs further include instructions for: determining that an instance of non-compliance to a second surgical protocol associated with a second surgical milestone has occurred in the operating room; and calculating the audit score for the surgery based on the instance of non-compliance to the first surgical protocol and the instance of non-compliance to the second surgical protocol.

Optionally, the audit score is based on a weighted calculation of the instance of non-compliance to the first surgical protocol and the instance of non-compliance to the second surgical protocol.

Optionally, the one or more programs further include instructions for: comparing the audit score against a predefined audit score threshold.

Optionally, the predefined audit score threshold is associated with a type of the surgery in the operating room.

Optionally, the one or more programs further include instructions for: displaying the audit score on a display; and saving the audit score to an electronic medical record.

Optionally, the one or more programs further include instructions for: identifying a change to the surgical protocol; and outputting a recommendation based on the identified change to the surgical protocol.

Optionally, identifying a change to the surgical protocol comprises: identifying a correlation between an outcome of the surgery in the operating room and the instance of non-compliance to the surgical protocol.

Optionally, the one or more programs further include instructions for: recommending retraining of the surgical protocol based on the instance of non-compliance to the surgical protocol.

Optionally, the one or more programs further include instructions for: determining an identity or a surgical function of a person associated with the instance of non-compliance; and determining whether to recommend a change to the surgical protocol or to recommend retraining of the surgical protocol at least partially based on the identity or the surgical function of the person associated with the instance of non-compliance.

Optionally, the first set of one or more trained machine-learning models is the same as the second set of one or more trained machine-learning models.

Optionally, the first set of one or more trained machine-learning models is different from the second set of one or more trained machine-learning models.

Optionally, the one or more activities include: linen changing on a surgical table; cleaning of the surgical table; wiping of the surgical table; application of a disinfectant; introduction of a surgical equipment; preparation of the surgical equipment; entrance of a person into the operating room; exiting of the person out of the operating room; opening of a door in the operating room; closing of the door in the operating room; donning of surgical attire; contamination of sterile instruments; contact between anything sterile and a non-sterile surface; preparation of a patient; usage of one or more blood units; usage of one or more surgical sponges; usage of one or more surgical swabs; collection and/or disposal of waste; fumigation; sterile zone violation; a conducted time-out; a conducted debriefing; fogging; or any combination thereof.

Optionally, the second set of one or more trained machine-learning models is configured to detect and/or track one or more objects in the operating room.

Optionally, the one or more objects include: one or more surgical tables; one or more surgical lights; one or more cleaning supplies; one or more disinfectants; one or more linens; one or more surgical equipment; one or more patients; one or more medical staff members; attire of the one or more medical staff members; one or more doors in the operating room; one or more blood units; one or more surgical sponges; one or more surgical swabs; or any combination thereof.

Optionally, the attire of the one or more medical staff members includes: a surgical mask, a surgical cap, a surgical glove, a surgical gown, or any combination thereof.

Optionally, the one or more surgical equipment includes: one or more imaging devices, one or more monitoring devices, one or more surgical tools, or any combination thereof.

Optionally, the one or more programs further include instructions for: calculating a ratio between medical staff members and patients in the operating room.

Optionally, detecting the surgical milestone comprises: obtaining, from the first set of one or more trained machine-learning models, one or more detected objects or events; and determining, based upon the one or more detected objects or events, the surgical milestone.

Optionally, the one or more programs further include instructions for determining that the severity level meets a predefined severity threshold, and in accordance with the determination that the determined severity level meets the predefined severity threshold starting an intervention. The method may further comprise: determining that the severity level does not meet the predefined severity threshold, and in accordance with the determination that the severity level does not meet the predefined severity threshold foregoing starting the intervention.

Optionally, the intervention is configured for drawing attention to meeting of the severity level. Optionally, the system may alert or notify the OR team of the severe protocol infraction. The alert may be visual, auditory, haptic, or any combination thereof. The alert may comprise an indication on one or more OR displays and/or one or more OR dashboards. The alert may optionally comprise a video or a textual description of the detected infraction and/or how severe it is. Optionally, the intervention is configured for at least momentarily pausing an operation. Optionally, the intervention comprises one or more of: altering the OR lighting, such as dimming the lights or setting the lights to higher brightness; modifying a view on a monitor, such as blocking or blurring the view, e.g. a camera view; blocking or providing haptic feedback on controls, e.g. of a surgical robot; blocking or providing, e.g. haptic, feedback on medical equipment, such as a diagnostic imaging device, an anesthesia machine, a staple gun, a retractor, a clamp, an endoscope, an electrocautery tool; or the like.

An exemplary non-transitory computer-readable storage medium stores one or more programs, the one or more programs comprising instructions, which when executed by one or more processors of an electronic device, cause the electronic device to perform any of the methods described herein.

### BRIEF DESCRIPTION OF THE FIGURES

FIG. 1 illustrates an exemplary view of a medical care area.
FIG. 2 illustrates an exemplary process for determining non-compliance to surgical protocols in an operating room.
FIG. 3A illustrates an exemplary machine-learning model used to detect surgical milestones.
FIG. 3B illustrates an exemplary machine-learning model used to detect objects and/or events, which are in turn used to detect surgical milestones.
FIG. 4A illustrates an exemplary machine-learning model used to detect compliant and/or non-compliant activities.
FIG. 4B illustrates an exemplary machine-learning model used to detect objects and/or events, which are in turn used to detect compliant and/or non-compliant activities.
FIG. 5 illustrates an exemplary electronic device.

### DETAILED DESCRIPTION

Disclosed herein are exemplary devices, apparatuses, systems, methods, and non-transitory storage media for determining non-compliance to surgical protocols in an operating room. Examples of the present disclosure can automate auditing of surgical workflows and improve the efficiency and accuracy of the audit logs. Some examples of the present disclosure include a system that employs a plurality of cameras in an operating room and processes live video streams using machine-learning algorithms such as object detection and tracking techniques to detect (e.g., in real time) instances of non-compliance when required protocols have been violated. In some examples, the machine-learning algorithms can be trained to monitor activities in the surgical workflow and recognize: adherence to operating room preparation and turnover protocols, non-compliance with sterile protocol, non-compliance with surgical attire, etc. The system can provide alerts in real time for certain protocol violations to prevent SSIs. Alternatively, or additionally, the system can provide interventions in real time for certain protocol violations to prevent SSIs. Additionally, the system can provide suggestions for retraining opportunities and protocol enhancements. Accordingly, examples of the present disclosure provide an efficient and accurate mechanism for conducting surgical audits and improve surgical safety and patient outcomes in ongoing and future surgeries.

The following description sets forth exemplary methods, parameters, and the like. It should be recognized, however, that such description is not intended as a limitation on the scope of the present disclosure but is instead provided as a description of exemplary examples.

Although the following description uses terms "first," "second," etc. to describe various elements, these elements should not be limited by the terms. These terms are only used to distinguish one element from another. For example, a first graphical representation could be termed a second graphical representation, and, similarly, a second graphical representation could be termed a first graphical representation, without departing from the scope of the various described examples. The first graphical representation and the second graphical representation are both graphical representations, but they are not the same graphical representation.

The terminology used in the description of the various described examples herein is for the purpose of describing particular examples only and is not intended to be limiting. As used in the description of the various described examples and the appended claims, the singular forms "a," "an," and "the" are intended to include the plural forms as well, unless the context clearly indicates otherwise. It will also be understood that the term "and/or" as used herein refers to and encompasses any and all possible combinations of one or more of the associated listed items. It will be further understood that the terms "includes," "including," "comprises," and/or "comprising," when used in this specification, specify the presence of stated features, integers, steps, operations, elements, and/or components, but do not preclude the presence or addition of one or more other features, integers, steps, operations, elements, components, and/or groups thereof.

The term "if' is, optionally, construed to mean "when" or "upon" or "in response to determining" or "in response to detecting," depending on the context. Similarly, the phrase "if it is determined" or "if [a stated condition or event] is detected" is, optionally, construed to mean "upon determining" or "in response to determining" or "upon detecting [the stated condition or event]" or "in response to detecting [the stated condition or event]," depending on the context.

Certain aspects of the present disclosure include process steps and instructions described herein in the form of an algorithm. It should be noted that the process steps and instructions of the present disclosure could be embodied in software, firmware, or hardware and, when embodied in software, could be downloaded to reside on and be operated from different platforms used by a variety of operating systems. Unless specifically stated otherwise as apparent from the following discussion, it is appreciated that, throughout the description, discussions utilizing terms such as "processing," "computing," "calculating," "determining," "displaying," "generating" or the like, refer to the action and processes of a computer system, or similar electronic computing device, that manipulates and transforms data represented as physical (electronic) quantities within the computer system memories or registers or other such information storage, transmission, or display devices.

The present disclosure in some examples also relates to a device for performing the operations herein. This device may be specially constructed for the required purposes, or it may comprise a general purpose computer selectively activated or reconfigured by a computer program stored in the computer. Such a computer program may be stored in a non-transitory, computer readable storage medium, such as, but not limited to, any type of disk, including floppy disks, USB flash drives, external hard drives, optical disks, CD-ROMs, magnetic-optical disks, read-only memories (ROMs), random access memories (RAMs), EPROMs, EEPROMs, magnetic or optical cards, application specific integrated circuits (ASICs), or any type of media suitable for storing electronic instructions, and each coupled to a computer system bus. Furthermore, the computers referred to in the specification may include a single processor or may be architectures employing multiple processor designs for increased computing capability.

The methods, devices, and systems described herein are not inherently related to any particular computer or other apparatus. Various general-purpose systems may also be used with programs in accordance with the teachings herein, or it may prove convenient to construct a more specialized apparatus to perform the required method steps. The required structure for a variety of these systems will appear from the description below. In addition, the present invention is not described with reference to any particular programming language. It will be appreciated that a variety of programming languages may be used to implement the teachings of the present invention as described herein.

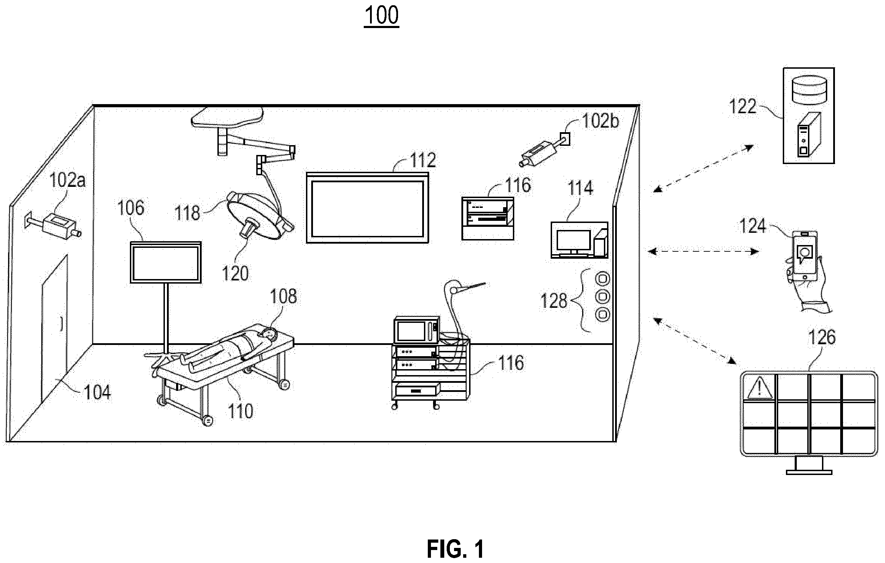

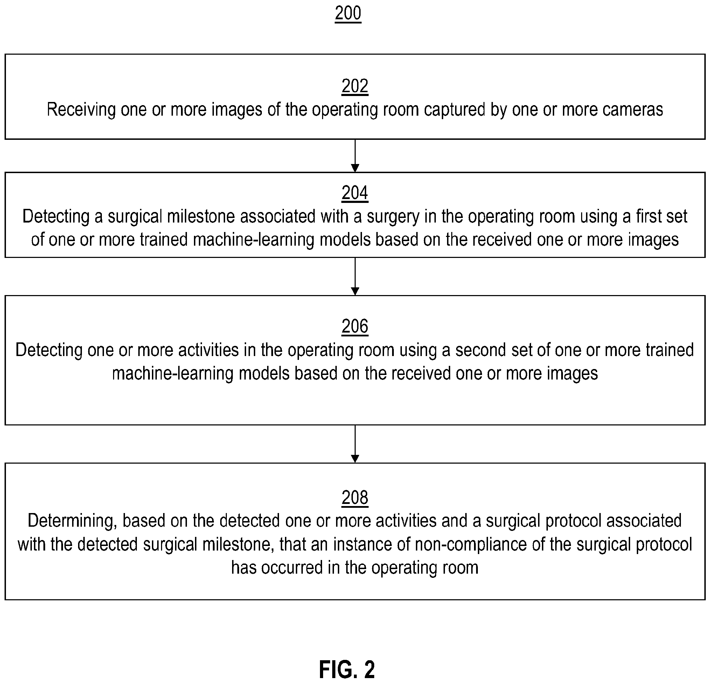

FIG. 1 illustrates an exemplary view of a medical care area 100. In the illustrated example, the medical care area 100 is an operating room where surgical operations are carried out in an antiseptic environment. The medical care area 100 includes one or more doors such as door 104, one or more medical charts, such as chart 106, one or more case carts, a patient, such as patient 108, an operating table, such as operating room table 110, one or more operating room monitors, such as operating room monitor 112, one or more computer systems, such as computer system 114, one or more pieces of medical equipment, such as medical equipment 116, one or more surgical lights, such as surgical light 118, one or more cameras, such as cameras 102a, 102b, and 120, and one or more sensors, such as sensors 128 (e.g., for monitoring various environmental factors. Inside or outside operating room 100, there may be one or more electronic medical record systems (EMR systems), such as electronic medical record system 122, one or more mobile devices, such as mobile device 124, and one or more displays, such as display 126. It should be understood that the aforementioned list is exemplary and there may be different, additional or fewer items in or associated with the operating room. For instance, the medical care area may include multiple doors, for example, a door that connects to a sterile room where sterile equipment and staff enter/exit through and another door that connects to a non-sterile corridor where patient enters/exits through. Additional exemplary objects that may be found in operating room 100 are provided with reference to FIG. 2.

The cameras (e.g., cameras 102a, 102b, and 120) can be oriented toward one or more areas or objects of interest in the operating room. For example, one or more cameras can be oriented toward: the door such that they can capture images of the door, the operating table such that they can capture images of the operating table, the patient such that they can capture images of the patient, medical equipment (e.g., X-Ray device, anesthesia machine, staple gun, retractor, clamp, endoscope, electrocautery tool, fluid management system, waste management system, suction units, etc.) such that they can capture images of the medical equipment, surgical staff (e.g., surgeon, anesthesiologist, surgical assistant, scrub nurse, circulating nurse, registered nurse) such that they can capture images of the surgical staff, etc. Multiple cameras may be placed in different locations in the operating room such that they can collectively capture a particular area or object of interest from different perspectives. Some cameras can be configured to track a moving object. The one or more cameras can include PTZ cameras. The cameras can include cameras that can provide a video stream over a network. The one or more cameras can include a camera integrated into a surgical light in the operating room.

An aspect of the present disclosure is to automate auditing of surgical workflows in a surgical environment (e.g., operating room 100) while improving the efficiency and accuracy of the audit logs. An example includes a system that employs a plurality of cameras in an operating room and processes live video streams using machine-learning algorithms such as object detection and tracking techniques to detect (e.g., in real time) instances of non-compliance when required protocols have been violated. The machine-learning algorithms can be trained to monitor activities in the surgical workflow and recognize: adherence to operating room preparation and turnover protocols, non-compliance with sterile protocol, non-compliance with surgical attire, inadvertent or accidental contamination, etc. The system can provide alerts and/or interventions in real time for certain protocol violations to prevent SSIs and/or track instances of non-compliance in audit logs and for downstream statistical analysis. For example, alerts and/or interventions can be useful in cases of inadvertent or accidental contamination without awareness to correct user behavior in real time and/or to proactively prevent SSIs before they occur. If contamination has occurred, corrective action can be taken to prevent the spread of undesirable microbes or contaminants. Tracking instances of non-compliance can be useful in cases of inadvertent or accidental contamination with awareness. Additionally, the system can provide suggestions for retraining opportunities and protocol enhancements. Accordingly, examples of the present disclosure provide an efficient and accurate mechanism for conducting surgical audit, while improving surgical safety and patient outcomes in ongoing and future surgeries.

FIG. 2 illustrates an exemplary process 200 for determining non-compliance to surgical protocols in an operating room. Process 200 is performed, for example, using one or more electronic devices implementing a software platform. In this example, process 200 is performed using a client-server system, and the blocks of process 200 are divided up in any manner between the server and a client device. Alternatively, the blocks of process 200 may be divided up between the server and multiple client devices. Alternatively, process 200 may be performed using only a client device or only multiple client devices. In process 200, some blocks are, optionally, combined, the order of some blocks is, optionally, changed, and some blocks are, optionally, omitted. Additional steps may be performed in combination with the process 200. Accordingly, the operations as illustrated (and described in greater detail below) are exemplary by nature and, as such, should not be viewed as limiting.

At block 202, an exemplary system (e.g., one or more electronic devices) can receive one or more images of the operating room captured by one or more cameras (e.g., cameras 102a and/or 102b in FIG. 1). The one or more images may include video frames. Alternatively, or additionally, the one or more images may include still images. As discussed above, the one or more cameras can be placed inside the operating room. The one or more cameras can be oriented toward: the door such that they can capture images of the door, the operating table such that they can capture images of the operating table, medical equipment (e.g., diagnostic imaging device, anesthesia machine, staple gun, retractor, clamp, endoscope, electrocautery tool) such that they can capture images of the medical equipment, surgical staff (e.g., surgeon, anesthesiologist, surgical assistant, nurse) such that they can capture images of the surgical staff, etc. Multiple cameras can be placed in different locations in the operating room such as they can collectively capture a particular area or object of interest from different perspectives. The one or more cameras can include PTZ cameras. The one or more cameras can include a camera integrated into a surgical light in the operating room.

Multiple cameras may be placed at different angles oriented toward a first door (e.g., a door the patient enters through) and/or a second door (e.g., a door sterile equipment and staff enter through) in the operating room, multiple cameras may be oriented toward the operating table from different angles, one or more cameras may be oriented toward the surgical lights and the surgeon, and one or more cameras may be oriented toward the surgical support staff. Different cameras, depending on the orientation of the camera, may be associated with different models configured to detect different objects such that images captured by a given camera are processed by associated model(s), as described in detail below.

The one or more images can include images captured by one or more surgical devices (e.g., endoscopes). By utilizing images captured by cameras generally installed in the operating room in conjunction with information from surgical devices, the system may provide a more accurate and realistic identification of surgical milestones and activities in blocks 204 and 206.

At block 204, the system can detect a surgical milestone associated with a surgery in the operating room using a first set of one or more trained machine-learning models based on the received one or more images. The system can be configured to determine a plurality of surgical milestones, which are described in detail herein. A milestone may refer to a phase or period of time during a surgical workflow (e.g., surgical phase), or a specific time point during the surgical workflow. A surgical milestone can refer to a preoperative activity, an intraoperative activity, or a postoperative activity, as discussed herein. Some surgical milestones may include specific steps (e.g., making an incision, removing an organ) of a surgery.

A surgical milestone can indicate the stage of progression through a surgical procedure or a surgical workflow. The plurality of predefined milestones can include: whether an operating room is ready, whether operating room setup has started, whether a medical staff member (e.g., the surgeon, the scrub nurse, the technician) is donning surgical attire (e.g., masks, gloves, caps, gowns), whether operating room equipment is being set up, whether the patient is brought in to the operating room, whether the patient is ready for intubation or anesthesia, whether a timeout is occurring, whether the timeout has occurred, whether the patient is intubated or anesthetized, whether the patient has been prepped and draped for surgery, whether the patient is ready for surgery, whether a surgery site prep is complete, whether a surgery has started, whether the surgery is closing, whether a dressing is applied to the patient, whether the surgery is stopped, whether the patient is brought out of the operating room, whether the operating room is being cleaned, whether the operating room is clean, or any combination thereof. It should be understood that the foregoing list of milestones is merely exemplary. There may be fewer, additional, or different predefined milestones, for instance, depending on a type of surgical procedure.

The system can be configured to use the one or more trained machine learning models to detect one or more detected objects or events, which are in turn used to determine the one or more surgical milestones (e.g., surgical time points, surgical phases). The one or more trained machine learning models can include an object detection algorithm, an object tracking algorithm, a video action detection algorithm, an anomaly detection algorithm, or any combination thereof.

The system can be configured to first use an object detection algorithm to detect a particular type of object in an image, and then use an object tracking algorithm to track the movement and/or status of the detected object in subsequent images. Using one or more object detection algorithms, the system may detect one or more objects and assign an object ID to each detected object. The one or more object detection algorithms can comprise machine-learning models such as a 2D convolutional neural network (CNN) or 3D-CNN (e.g., MobileNetV2, ResNet, MobileNetV3, CustomCNN). After the objects are detected, the system may then use one or more object tracking algorithms to track the movement of the detected objects. The one or more object tracking algorithms can comprise any computer-vision algorithms for tracking objects and can comprise non-machine-learning algorithms. The object tracking algorithm(s) may involve execution of more lightweight code than the object detection algorithm(s), thus improving efficiency and reducing latency for surgical milestone determination. An object detection algorithm may include an instance segmentation algorithm, which can be configured to simultaneously perform classification (e.g., determining what type of object an image depicts), semantic segmentation (e.g., determining what pixels in the image belong to the object), and instance association (e.g., identifying individual instances of the same class; for example, person1 and person2). Additionally, in real-world scenes, a given visual object may be occluded by other objects. Although human vision systems can locate and recognize severely occluded objects with temporal context reasoning and prior knowledge, it may be challenging for classical video understanding systems to perceive objects in the heavily occluded video scenes. Accordingly, some examples include machine-learning algorithms that take into account the temporal component of the video stream. For example, the system may perform spatial feature calibration and temporal fusion for effective one-stage video instance segmentation. As another example, the system may perform spatio-temporal contrastive learning for video instance segmentation. Additional information on these exemplary algorithms can be found, for example, in Li et al., "Spatial Feature Calibration and Temporal Fusion for Effective One-stage Video Instance Segmentation", arXiv:2104.05606v1, available at https://doi.org/10.48550/arXiv.2104.05606, and Jiang et al., "STC: Spatio-Temporal Contrastive Learning for Video Instance Segmentation", arXiv:2202.03747v1, available at https://doi.org/10.48550/arXiv.2202.03747, both of which are incorporated herein by reference.

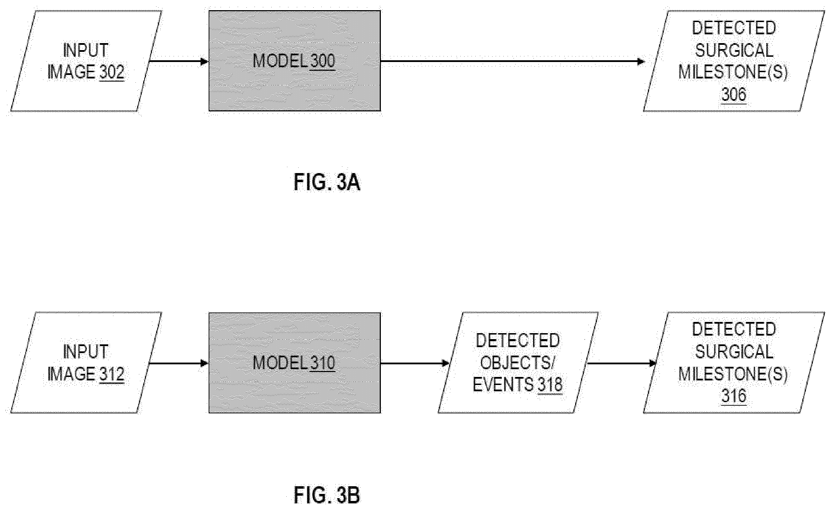

The tracked movement and/or status of one or more detected objects can then be used to determine events occurring in the operating room. For example, the system can first use an object detection model to detect a stretcher in an image and then use an object tracking algorithm to detect when the stretcher crosses door coordinates to determine that the stretcher is being moved into the operating room (i.e., an event). The one or more trained machine-learning models can be trained using a plurality of annotated images (e.g., annotated with labels of object(s) and/or event(s)). Further description of such machine learning models is provided below with reference to FIG. 3A.

An object that the system can detect can include physical items, persons, or parts thereof, located inside, entering, or leaving an operating room. The object can for example include a stretcher, a patient, a surgeon, an anesthesiologist, the surgeon's hand, a surgical assistant, a scrub nurse, a technician, a nurse, a scalpel, sutures, a staple gun, a door to a sterile room, a door to a non-sterile corridor, a retractor, a clamp, an endoscope, an electrocautery tool, an intubation mask, a surgical mask, a C-Arm, an Endoscopic Equipment Stack, an anesthesia machine, an anesthesia cart, a fluid management system, a waste management system, a waste disposal receptacle, an operating table, surgical table accessories, an equipment boom, an anesthesia boom, an endoscopic equipment cart, surgical lights, a case cart, a sterile back table, a sterile mayo stand, a cleaning cart, an X-Ray device, an imaging device, a trocar, a surgical drape, operating room floor, EKG leads, ECG leads, bed linens, a blanket, a heating blanket, a lap belt, safety straps, a pulse oximeter, a blood pressure machine, an oxygen mask, an IV, or any combination thereof.

An event that the system can detect can include a status, change of status, and/or an action associated with an object. The event can for example include whether the surgical lights are turned off, whether the operating table is vacant, whether the bed linens are wrinkled, whether the bed linens are stained, whether the operating table is wiped down, whether a new linen is applied to the operating table, whether a first sterile case cart is brought into the operating room, whether a new patient chart is created, whether instrument packs are distributed throughout the operating room, whether booms and suspended equipment are repositioned, whether the operating table is repositioned, whether a nurse physically exposes instrumentation by unfolding linen or paper, or opening instrumentation containers using a sterile technique, whether the scrub nurse entered the operating room, whether the technician entered the operating room, whether the scrub nurse is donning a gown, whether the circulating nurse is securing the scrub nurse's gown, whether the scrub nurse is donning gloves using the sterile technique, whether the sterile back table or the sterile mayo stand is being set with sterile instruments, whether the patient is wheeled into the operating room on a stretcher, whether the patient is wheeled into the operating room on a wheel chair, whether the patient walked into the operating room, whether the patient is carried into the operating room, whether the patient is transferred to the operating table, whether the patient is covered with the blanket, whether the lap belt is applied to the patient, whether the pulse oximeter is placed on the patient, whether the EKG leads are applied to the patient, whether the ECG leads are applied to the patient, whether the blood pressure cuff is applied to the patient, whether a surgical sponge and instrument count is conducted, whether a nurse announces a timeout, whether a surgeon announces a timeout, whether an anesthesiologist announces a timeout, whether activities are stopped for a timeout, whether the anesthesiologist gives the patient the oxygen mask, whether the patient is sitting and leaning over with the patient's back cleaned and draped, whether the anesthesiologist inspects the patient's anatomy with a long needle, whether the anesthesiologist injects medication into the patient's back, whether the anesthesiologist indicates that the patient is ready for surgery, whether the patient is positioned for a specific surgery, whether required surgical accessories are placed on a table, whether padding is applied to the patient, whether the heating blanket is applied to the patient, whether the safety straps are applied to the patient, whether a surgical site on the patient is exposed, whether the surgical lights are turned on, whether the surgical lights are positioned to illuminate the surgical site, whether the scrub nurse is gowning the surgeon, whether the scrub nurse is gloving the surgeon, whether skin antiseptic is applied, whether the surgical site is draped, whether sterile handles are applied to the surgical lights, whether a sterile team member is handing off tubing to a non-sterile team member, whether a sterile team member is handing off electrocautery to a non-sterile team member, whether the scalpel is handed to the surgeon, whether an incision is made, whether the sutures are handed to the surgeon, whether the staple gun is handed to the surgeon, whether the scrub nurse is handing a sponge to a sponge collection basin, whether an incision is closed, whether dressing is applied to cover a closed incision, whether the surgical lights are turned off, whether the anesthesiologist is waking the patient, whether the patient is returned to a supine position, whether extubation is occurring, whether instruments are being placed on the case cart, whether a garbage bag is being tied up, whether the bed linens are collected and tied up, whether the operating table surface is cleaned, whether the operating room floor is being mopped, whether the patient is being transferred to a stretcher, whether the patient is being brought out of the operating room, whether the surgical table is dressed with a clean linen, whether a second sterile case cart is brought into the operating room, or any combination thereof.

Instead of using trained machine-learning models to detect objects/events (which are then used to determine surgical milestones), the system may use trained machine-learning models to output surgical milestones directly. A trained machine-learning model of the one or more trained machine-learning models can be a machine-learning model (e.g., deep-learning model) trained using annotated surgical video information, where the annotated surgical video information includes annotations of at least one of the plurality of predefined surgical milestones. Further description of such machine learning models is provided below with reference to FIG. 3B.

The system may perform a spatial analysis (e.g., based on object detection/tracking as discussed above), a temporal analysis, or a combination thereof. The system may perform the temporal analysis using a temporal deep neural network (DNN), such as LSTM, Bi-LSTM, MSTCN, etc. The DNN may be trained using one or more training videos in which the start time and the end time of various surgical milestones are bookmarked. The temporal analysis may be used to predict remaining surgery duration, as discussed below.

The one or more trained machine-learning models used herein can comprise a trained neural network model, such as a 2D CNN, 3D-CNN, temporal DNN, etc. For example, the models may comprise ResNet50, AlexNet, Yolo, I3D ResNet 50, LSTM, MSTCN, etc. The one or more trained machine-learning models may comprise supervised learning models that are trained using annotated images such as human-annotated images. Additionally or alternatively, the one or more trained machine-learning model may comprise self-supervised learning models where a specially trained network can predict the remaining surgery duration, without relying on labeled images. As examples, a number of exemplary models are described in G. Yengera et al., "Less is More: Surgical Phase Recognition with Less Annotations through Self-Supervised Pre-training of CNN-LSTM Networks," arXiv:1805.08569 [cs.CV], available at https://arxiv.org/abs/1805.08569. For example, an exemplary model may utilize a self-supervised pre-training approach based on the prediction of remaining surgery duration (RSD) from laparoscopic videos. The RSD prediction task is used to pre-train a CNN and long short-term memory (LSTM) network in an end-to-end manner. The model may utilize all available data and reduces the reliance on annotated data, thereby facilitating the scaling up of surgical phase recognition algorithms to different kinds of surgeries. Another example model may comprise an end-to-end trained CNN-LSTM model for surgical phase recognition. It should be appreciated by one of ordinary skill in the art that other types of object detection algorithms, object tracking algorithms, video action detection algorithms, that provide sufficient performance and accuracy (e.g., in real time) can be used. The system can include machine-learning models associated with a family of architectures based on visual transformers, which may perform image recognition at scale. An exemplary framework is a Self-supervised Transformer with Energy-based Graph Optimization (STEGO) and may be capable of jointly discovering and segmenting objects without any human supervision. Building upon another self-supervised architecture, DINO, STEGO can distill pre-trained unsupervised visual features into semantic clusters using a novel contrastive loss. Additional information on visual transformers can be found, for example, in Caron et al., "An Image is Worth 16x16 Words: Transformers for Image Recognition at Scale", arXiv:2010.11929v2, available at https://doi.org/10.48550/arXiv.2010.11929, which is incorporated herein by reference. Additional information on DINO and STEGO can be found, for example, in Hamilton et al., "Unsupervised Semantic Segmentation by Distilling Feature Correspondences", arXiv:2203.08414v1, available at https://doi.org/10.48550/arXiv.2203.08414, and Caron et al., "Emerging Properties in Self-Supervised Vision Transformers", arXiv:2104.14294v2, available at https://doi.org/10.48550/arXiv.2104.14294, which are incorporated herein by reference. Additional details related to detection of surgical milestones can be found in U.S. Provisional Application entitled "SYSTEMS AND METHODS FOR MONITORING SURGICAL WORKFLOW AND PROGRESS" (Attorney Docket No.: 16890-30044.00), which is incorporated herein by reference.

At block 206, the system can detect one or more activities in the operating room using a second set of one or more trained machine-learning models based on the received one or more images. The activities may be monitored for the purpose of detecting non-compliance with one or more surgical protocols. For example, the models may be configured to monitor any activities from which compliance and/or non-compliance to specific requirements in a surgical protocol can be detected.

As an example, a surgical protocol may comprise one or more requirements related to preparation of the surgery before a surgery commences. The protocol may require that linens be changed on the surgical table in the operating room, that various objects in the operating room (e.g., surgical table, surgical lights, equipment) be cleaned, wiped, and/or disinfected, that the necessary equipment and instruments (e.g., major imaging equipment) are available for the surgery, that the patient is properly prepared, etc. Accordingly, the second set of one or more machine-learning models may be configured to detect activities such as: change of linen on the surgical table, cleaning/wiping/disinfection of objects in the operating room, availability of equipment necessary for the surgery, patient preparation performed before the surgery starts, availability of routine monitoring equipment (e.g., pulse oximeter for monitoring pulse and O₂ saturation in the blood stream, EKG/ECG heart monitor, automatic blood pressure machine), etc. Additionally or alternatively, the second set of one or more machine-learning models may be configured to detect the lack of such activities in the operating room. Metadata related to the activities (time stamps, duration, count) may also be obtained based on the output of the machine-learning models.

As another example, a surgical protocol may comprise one or more requirements related to intraoperative surgical safety. The protocol may comprise specific requirements related to the traffic in the operating room, the opening and closing of doors in the operating room (e.g., permissible counts of door openings and door closings, permissible durations of door(s) being open and closing, aggregate duration of door(s) being open), proper surgical attire, proper activities in the sterile zone during the surgery, equipment and instruments introduced into the operating room during the surgery, contamination of sterile equipment and instruments, use of blood units, use of monitoring equipment, use of sponges and swabs, etc. Accordingly, the second set of one or more machine-learning models may be configured to detect activities such as: staff members moving in/out of the operating room during the surgery, when each door in the operating room opens or closes during the surgery, surgical attire by the staff member (surgical mask, surgical cap, surgical gloves, body hair concealed, etc.), people entering and exiting sterile zone during the surgery, any equipment brought into the operating room during the surgery, contamination of sterile instruments and equipment during the surgery, blood units used, blood units required, sponges and swabs used, etc. Additionally or alternatively, the second set of one or more machine-learning models may be configured to detect the lack of such activities in the operating room. Metadata related to the activities (time stamps, duration, count) may also be obtained based on the output of the machine-learning models.

As another example, a surgical protocol may comprise one or more requirements related to cleaning and disinfection. The protocol may comprise specific requirements related to cleaning activities, fumigation, fogging, collection and disposal of bio-waste post-surgery, collection and disposal of sharps, emptying garbage receptacles, mopping the floor, wiping down walls, changing of table linen, attending to (e.g., emptying and cleaning of) fluid management systems, removal of fluid management systems from the OR for preparation for the next surgery, etc. Accordingly, the second set of one or more machine-learning models may be configured to detect activities such as: cleaning, fumigation, fogging, application of disinfection chemicals to equipment, collection and disposal of operating room swabs, etc. Additionally or alternatively, the second set of one or more machine-learning models may be configured to detect the lack of such activities in the operating room. Metadata related to the activities (time stamps, duration, count of an activity) may also be obtained based on the output of the machine-learning models.

As another example, a surgical protocol may comprise one or more requirements related to operational parameters of the surgery. For example, the protocol may require that the nurse to patient ratio to be lower than a threshold. As another example, the protocol may require that the number of times a door in the operating room is opened during the surgery is lower than a threshold. The output of the second set of one or more machine-learning models may be used to calculate the operational parameters for each surgery.

It should be appreciated the activities described above are merely exemplary. The second set of machine-learning model can be configured to detect any activities from which compliance and/or non-compliance to specific requirements in a surgical protocol can be detected. The one or more activities for example include: linen changing on a surgical table; cleaning of the surgical table; wiping of the surgical table; application of a disinfectant; introduction of a surgical equipment; preparation of the surgical equipment; entrance of a person into the operating room; exiting of the person out of the operating room; opening of a door in the operating room; closing of the door in the operating room; donning of surgical attire; contamination of sterile instruments; contact between anything sterile and a non-sterile surface (e.g., an inadvertent contact from the surgeons glove with a non-sterile surface of the surgical light while using the sterile control interface of the light); preparation of a patient; usage of one or more blood units; usage of one or more surgical sponges; usage of one or more surgical swabs; collection and/or disposal of waste; fumigation; sterile zone violation (e.g., suspension or transfer of anything non-sterile above (within the 3D space above) the surgical site); a conducted time-out; a conducted debriefing; fogging; or any combination thereof.

A surgeon's technical skills assessment can also be a subject of audit and can be evaluated using various machine learning models. For example, a trained machine-learning model can receive information related to a surgeon (e.g., videos of the surgeon's procedures) and provide one or more outputs indicative of the surgeon's technical skill level. Exemplary techniques for assessing a surgeon's technical skills can be found, for example, in Lam et al., "Machine learning for technical skill assessment in surgery: a systematic review", Digit. Med. 5, 24 (2022), which is incorporated herein by reference. The assessment of the surgeon's technical skills as provided by a machine-learning model may be incorporated in the calculation of the audit score described below.

The system may be configured to invoke different machine-learning models depending on the current surgical milestone and/or a type of the surgery. For example, if the system determines (e.g., in block 204) that the operating room is being prepared for an upcoming surgery, which has not started, the system may invoke the machine-learning models for detecting potential non-compliant activities during pre-operation preparation, but not the machine-learning models for detecting potential non-compliant activities during a surgery, thereby improving efficiency and reducing computational demands. As another example, different surgeries may require different equipment; thus, depending on the type of the surgery, the system may invoke different machine-learning models for detecting necessary equipment for the type of the surgery.

In order to detect the activities, the system may use the one or more trained machine learning models to detect one or more detected objects, which are in turn used to determine the one or more activities. The one or more objects may include: one or more surgical tables; one or more surgical lights; one or more cleaning supplies; one or more disinfectants; one or more linens; one or more surgical equipment; one or more patients; one or more medical staff members; attire of the one or more medical staff members; one or more doors in the operating room; one or more blood units; one or more surgical sponges; one or more surgical swabs; or any combination thereof. The attire of the one or more medical staff members can include: a surgical mask, a surgical cap, a surgical glove, a surgical gown, or any combination thereof. The one or more surgical equipment can include: one or more imaging devices, one or more diagnostic devices, one or more monitoring devices, one or more surgical tools, or any combination thereof.

At least some of the first set of one or more trained machine-learning models can be the same as some of the second set of one or more trained machine-learning models. For example, the same machine-learning model may be used to detect and/or track a particular object (e.g., a door in the operating room) in blocks 204 and 206. As another example, the same model may be used to detect an event in 204 and an activity in block 206. In other examples, the first set of one or more trained machine-learning models may be different from the second set of one or more trained machine-learning models.

The system can use the one or more trained machine learning models to detect one or more detected objects and/or events, which are in turn used to determine the one or more activities. The one or more trained machine learning models can include an object detection algorithm, an object tracking algorithm, a video action detection algorithm, an anomaly detection algorithm, or any combination thereof.

The system can be configured to first use an object detection algorithm to detect a particular type of object in an image, and then use an object tracking algorithm to track the movement and/or status of the detected object in subsequent images. Using one or more object detection algorithms, the system may detect one or more objects and assign an object ID to each detected object. The one or more object detection algorithms can comprise machine-learning models such as a 2D convolutional neural network (CNN) or 3D-CNN (e.g., MobileNetV2, ResNet, MobileNetV3, CustomCNN). After the objects are detected, the system may then use one or more object tracking algorithms to track the movement of the detected objects. The one or more object tracking algorithms can comprise any computer-vision algorithms for tracking objects and can comprise non-machine-learning algorithms. In some examples, the object tracking algorithm(s) may involve execution of more lightweight code than the object detection algorithm(s), thus improving efficiency and reducing latency for activity determination.

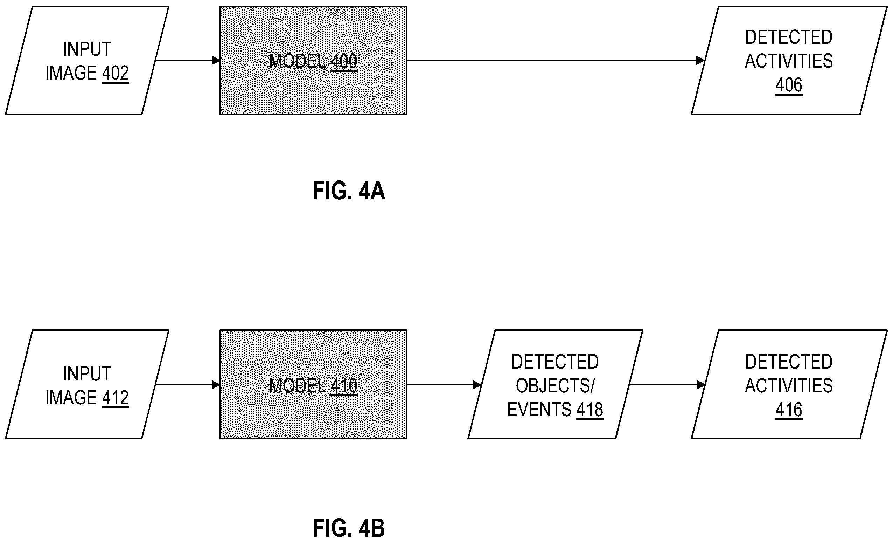

The tracked movement and/or status of one or more detected objects can then be used to determine events occurring in the operating room. For example, the system can first use an object detection model to detect a stretcher in an image and then use an object tracking algorithm to detect when the stretcher crosses door coordinates to determine that the stretcher is being moved into the operating room (i.e., an event). The one or more trained machine-learning models can be trained using a plurality of annotated images (e.g., annotated with labels of object(s) and/or event(s)). Further description of such machine learning models is provided below with reference to FIG. 4A.

Instead of using trained machine-learning models to detect objects/events (which are then used to determine activities), the system can use trained machine-learning models to output activities directly. A trained machine-learning model of the one or more trained machine-learning models can be a machine-learning model (e.g., deep-learning model) trained using annotated surgical video information, where the annotated surgical video information includes annotations of at least one of the plurality of predefined activities. Further description of such machine learning models is provided below with reference to FIG. 4B.

The system may perform a spatial analysis (e.g., based on object detection/tracking as discussed above), a temporal analysis, or a combination thereof. The system may perform the temporal analysis using a temporal deep neural network (DNN), such as LSTM, Bi-LSTM, MSTCN, etc. The DNN may be trained using one or more training videos in which the start time and the end time of various activities are bookmarked.

The one or more trained machine-learning models used herein can comprise a trained neural network model, such as a 2D CNN, 3D-CNN, temporal DNN, etc. For example, the models may comprise ResNet50, AlexNet, Yolo, I3D ResNet 50, LSTM, MSTCN, etc. In some examples, as discussed herein, the one or more trained machine-learning models may comprise supervised learning models that are trained using annotated images such as human-annotated images. Additionally or alternatively, the one or more trained machine-learning model may comprise self-supervised learning models where a specially trained network can predict the remaining surgery duration, without relying on labeled images. As examples, a number of exemplary models are described in G. Yengera et al., "Less is More: Surgical Phase Recognition with Less Annotations through Self-Supervised Pre-training of CNN-LSTM Networks," arXiv: 1805.08569 [cs.CV], available at https://arxiv.org/abs/1805.08569. For example, an exemplary model may utilize a self-supervised pre-training approach based on the prediction of remaining surgery duration (RSD) from laparoscopic videos. The RSD prediction task is used to pre-train a CNN and long short-term memory (LSTM) network in an end-to-end manner. The model may utilize all available data and reduces the reliance on annotated data, thereby facilitating the scaling up of activity recognition algorithms to different kinds of surgeries. Another example model may comprise an end-to-end trained CNN-LSTM model for surgical phase recognition. It should be appreciated by one of ordinary skill in the art that other types of object detection algorithms, object tracking algorithms, video action detection algorithms, that provide sufficient performance and accuracy (e.g., in real time) can be used.

At block 208, the system can determine, based on the detected one or more activities and a surgical protocol associated with the detected surgical milestone, that an instance of non-compliance to the surgical protocol has occurred in the operating room. For example, the detected activities may indicate a lack of or improper linen changing on a surgical table; a lack of or improper cleaning of the surgical table; a lack of or improper wiping of the surgical table; a lack of or improper application of a disinfectant; a lack of or improper introduction of a surgical equipment; a lack of or improper preparation of the surgical equipment; improper entrance of a person into the operating room; improper exiting of the person out of the operating room; improper opening of a door in the operating room; improper closing of the door in the operating room; a lack of or improper donning of surgical attire; contamination of sterile instruments; contact between anything sterile and a non-sterile surface (e.g., an inadvertent contact from the surgeons glove with a non-sterile surface of the surgical light while using the sterile control interface of the light); a lack of or improper preparation of a patient; improper usage of one or more blood units; improper usage of one or more surgical sponges; improper usage of one or more surgical swabs; improper collection and/or disposal of waste; improper fumigation; sterile zone violation (e.g., suspension or transfer of anything non-sterile above (within the 3D space above) the surgical site); an improperly conducted time-out; an improperly conducted debriefing; or any combination thereof.

In order to determine whether an activity is non-compliant in block 206, the system may analyze the activity in light of the surgical protocol requirements specific to the surgical milestone detected in 204. Depending on the surgical milestone, the surgical protocol, or what is considered to be a non-compliant activity may differ. For example, a lack of mask wearing may be considered acceptable if it occurs after the surgery, but considered non-compliant if it occurs during the surgery. As another example, a door that stays open for an extended period of time may be considered acceptable if it occurs before the surgery, but considered non-compliant if it occurs during the surgery. As another example, the proper location for disposing used instruments, sponges, and swabs may differ depending on whether a surgery is ongoing or has concluded. As another example, it may be considered acceptable for a person to not wear gloves to enter the OR during surgical preparation, but a lack of glove wearing may be considered non-compliant if it occurs during surgery. As another example, it may be considered acceptable for a person to touch/position the surgical light from the light handle without gloves and without the application of the sterile handle cover during surgical preparation, but doing so without wearing sterile gloves may be considered non-compliant if it occurs during surgery. As another example, it may be considered acceptable for a person to not use sterile techniques to handle surgical instruments after completion of surgery, but not using sterile techniques may be considered non-compliant if it occurs during surgery. As another example, it may be considered acceptable for a person to enter and exit the sterile zone after the surgery, but doing so may be considered non-compliant if it occurs during surgery.

Detection of non-compliances can be performed as an anomaly detection task. For example, instead of first enumerating various adverse events and then developing models for detecting them, an anomaly detection model can be trained end to end on a variety of surgical workflows that are deemed compliant. Accordingly, the anomaly detection model can receive a surgical workflow and provide an output indicative of whether or how far the input surgical workflow deviates from a normal range. Using the anomaly detection model, any surgical workflow that is classified to fall outside the normal range can be flagged as anomalous and, as a result, could be a potential compliance violation. Digital Twin environments can be used to generate enough compliant data for training such models.

If non-compliance is detected, the system may intelligently select one or more follow-up actions to take from a plurality of potential follow-up actions. The plurality of potential follow-up actions may include, but are not limited to: outputting an alert on a dashboard (e.g., in the operating room, in a control room), sending a message (e.g., an email, a text message), logging the non-compliance in a database, updating a report, recommending training and retraining, recommending protocol changes, performing downstream analytics, etc. The alert may be auditory, graphical, textual, haptic, or any combination thereof. The plurality of potential follow-up actions may also include starting an intervention, such as: locking a door, altering the OR lighting, such as dimming the lights or setting the lights to high brightness; modifying a view on a monitor, such as blocking or blurring the view, e.g. a camera view; blocking or providing haptic feedback on controls of medical equipment, e.g. of a surgical robot; blocking or providing, e.g. auditory, graphical, textual and/or haptic, feedback on medical equipment, such as a diagnostic imaging device, an anesthesia machine, a staple gun, a retractor, a clamp, an endoscope, an electrocautery tool; etc.

The system can be configured to determine a severity level of the instance of non-compliance to the surgical protocol and determine which follow-up action(s) to take accordingly. Optionally, the system may alert or notify the OR team of the severe protocol infraction. The alert may be visual, auditory, haptic, or any combination thereof. The alert may comprise an indication on one or more OR displays and/or one or more OR dashboards. The alert may optionally comprise a video or a textual description of the detected infraction and/or how severe it is.

For example, certain instances of non-compliance may not be considered severe enough to warrant intervention and/or a real-time alert because the real-time alert may be disruptive to the surgical staff, whereas certain instances of non-compliance, such as contamination of a sterile instrument during the course of surgery (e.g., surgical staff inadvertently touching the sterile portion of the sterile instrument with a contaminated glove), need to be reported in real-time, and/or may warrant an intervention, to prevent increased risk of surgical site infections. Thus, the system can determine that the severity level meets a predefined severity threshold and, in accordance with the determination, generate an alert and/or start an intervention. The system can also determine that the severity level does not meet the predefined severity threshold and, in accordance with the determination, forego generating the alert or intervention or delay the generation of the alert until a later time. The system may nevertheless still record the detected instance of non-compliance in the audit logs or a database for downstream analysis.

Certain instances of non-compliance may not be considered severe enough to warrant an intervention because the intervention may be disruptive to the surgery, so an alert may be more appropriate. Thus, the system can determine whether the severity level meets a predefined severity threshold and, in accordance with a determination that the determined severity level meets the predefined severity threshold, start an intervention.

Certain instances of non-compliance may not be considered severe enough to warrant an audio alert because the audio alert may be disruptive to the surgery, so a different type of alert (e.g., visual alert) may be more appropriate. Thus, the system can determine whether the severity level meets a predefined severity threshold and, in accordance with a determination that the determined severity level meets the predefined severity threshold, generate an audio alert. In accordance with a determination that the determined severity level does not meet the predefined severity threshold, the system can forego generating the alert, generate a text alert, or delay the generation of the audio alert until a later time. The system may nevertheless still record the detected instance of non-compliance in the audit logs or a database for downstream analysis.

Non-compliance to a surgical protocol may affect an audit score for: a surgery, an individual or a group of individuals (e.g., a surgical team), an organization (e.g., a department, a hospital), or any combination thereof. An audit score can quantify the amount of deviation from one or more surgical protocols. The calculated audit score can be provided to a user (e.g., displayed on a dashboard) in real time. The calculated audit score can be stored as a part of an audit log in a database (e.g., HIS, EMR, an audit log) for downstream analysis, etc. The system can compare the audit score against a predefined audit score threshold to determine how well a surgery, an individual, a group of individuals, and/or an organization are observing surgical protocols. The predefined audit score threshold can be associated with a type of surgery in the operating room.

The system can calculate an audit score for the surgery based on detected instances of non-compliance to the surgical protocol. The system can be configured to aggregate multiple instances of non-compliance across multiple surgical milestones into a single audit score. For example, one or more surgical protocols may specify 20 requirements associated with operating room setup, 10 requirements associated with patient preparation, 30 requirements associated with cleaning and disinfection, etc. Non-compliance to each requirement may be associated with a sub-score. The sub-scores can be aggregated to calculate a single audit score across multiple surgical milestones. A first surgical milestone can be associated with a first surgical protocol and a second surgical milestone can be associated with a second surgical protocol, and the system can calculate the audit score for the surgery based on an instance of non-compliance to the first surgical protocol and the instance of non-compliance to the second surgical protocol. The audit score can be based on a weighted calculation of the instance of non-compliance to the first surgical protocol and the instance of non-compliance to the second surgical protocol.

Non-compliance to different requirements may be scored and/or weighted differently, with more severe instances of non-compliance weighted heavier. As examples, contamination of sterile equipment may be weighted heavier than improper disposal of a cotton swab; a longer period of door opening may be weighted heavier than a shorter period of door opening.

The scoring mechanism can be configurable by a user. For example, a user can set how a given instance of non-compliance is scored. For example, the user can assign different sub-scores to violations of different requirements. As another example, the user can also specify that the first time a particular requirement is violated is scored 0 (i.e., ignored), but the score increases as the number of instances of non-compliance to the particular requirement increases. The scoring mechanism can depend on the facility, the type of surgery (e.g., cardiothoracic and orthopedic surgeries may be associated with more severe scoring mechanisms because infection can be very detrimental to the patient), etc.

Based on detected non-compliance with surgical protocols, the system can identify a change to the surgical protocol and output a recommendation based on the identified change to the surgical protocol. The system can be configured to identify a change to the surgical protocol by identifying a correlation between an outcome of the surgery in the operating room and the instance of non-compliance to the surgical protocol in a database or audit logs. For example, if a strong correlation (e.g., above a predefined threshold) is identified between violation of a particular requirement and post-surgery infection, the system may recommend adding the particular requirement to a checklist to minimize the likelihood of violation and thus improve outcomes in future surgeries.

Protocol enhancement recommendations can be developed by utilizing Digital Twins technology. A digital twin can include a virtual representation - a true-to-reality simulation of physics and materials - of a real-world physical asset or system (e.g., an operating room), which is continuously updated. Digital Twins technology can be used to generate a virtual twin of an operating room to provide a safe environment to test the changes in system performance. It can also be used to generate training data for machine-learning models, such as the machine-learning models described herein. Additional details of the Digital Twins technology can be found, for example, in "What Is a Digital Twin?", available at https://blogs.nvidia.com/blog/2021/12/14/what-is-a-digital-twin/,which is incorporated by reference herein.

Based on detected non-compliance with surgical protocols, the system can recommend training or retraining of the surgical protocol. The system can be configured to determine an identity or a surgical function of a person associated with the instance of non-compliance; and determine whether to recommend a change to the surgical protocol or to recommend retraining of the surgical protocol at least partially based on the identity or the surgical function of the person associated with the instance of non-compliance. For example, if the system detects that a requirement is violated by multiple people across departments and/or organizations, the system may determine that a general update to the surgical protocol (e.g., new checklists) is needed to ensure that the requirement is observed. But, if the system detects that a requirement is violated repeatedly by a particular person (e.g., a particular surgeon, a particular nurse), a particular group of people (e.g., a particular surgical team), or people of the same surgical function (e.g., scrub nurses, circulating nurses), the system may determine that the person or the group of people needs to be retrained on the requirement. The system may determine a need for both protocol enhancement and retraining. The identify or the surgical function of the person may be identified using facial recognition techniques, RFID or GPS signals of a device associated with the person, the person's attire/actions, the hospital record/schedules, HIS/EMR databases, or any combination thereof.

FIGS. 3A and 3B illustrate exemplary machine-learning models that can be used to detect surgical milestone(s). Both models 300 and 310 can receive an input image (e.g., an image received in block 202). The model(s) 300 can be configured to directly output one or more surgical milestones depicted in the input image. In contrast, the model(s) 310 can be configured to output one or more detected objects or events 318, which in turn can be used by the system to determine one or more surgical milestones depicted in the input image. Models 300 and 310 are described in detail below.

With reference to FIG. 3A, a model 300 is configured to receive an input image 302 and directly output an output 306 indicative of one or more surgical milestones detected in the input image 302. The model 300 can be trained using a plurality of training images depicting the one or more surgical milestones. For example, the model 300 can be trained using a plurality of annotated training images. Each of the annotated images can depict a scene of an operating room and include one or more labels indicating surgical milestone(s) depicted in the scene. The plurality of annotated training images can comprise a video in which surgical milestones are bookmarked. At least some of the annotated images can be captured in the same operating room (e.g., operating room 100) for which the model will be deployed. During training, the model receives each image of the annotated images and provides an output indicative of detected surgical milestone(s). The output is compared against the labels associated with the image. Based on the comparison, the model 300 can be updated (e.g., via a backpropagation process).

With reference to FIG. 3B, a model 310 is configured to receive an input image 312 and output one or more detected objects and/or events 318 depicted in the input image 312. Based on the one or more detected objects and/or events 318, the system can determine, as output 316, one or more surgical milestones detected in the input image 312. The one or more machine learning models can be trained using a plurality of training images depicting the one or more objects and/or events. For example, the model 310 can be trained using a plurality of annotated training images. Each of the annotated images can depict a scene of an operating room and include one or more labels indicating objects and/or events depicted in the scene. At least some of the annotated images can be captured in the same operating room (e.g., operating room 100) for which the model will be deployed. During training, the model receives each image of the annotated images and provides an output indicative of one or more detected objects and/or events. The output is compared against the labels associated with the image. Based on the comparison, the model 300 can be updated (e.g., via a backpropagation process).

FIGS. 4A and 4B illustrate exemplary machine-learning models that can be used to detect surgical milestone(s), in accordance with some examples. Both models 400 and 410 can receive an input image (e.g., an image received in block 202). The model(s) 400 can be configured to directly output one or more activities 406 depicted in the input image. In contrast, the model(s) 410 can be configured to output one or more detected objects or events 418, which in turn can be used by the system to determine one or more activities depicted in the input image. Models 400 and 410 are described in detail below.

With reference to FIG. 4A, a model 400 is configured to receive an input image 402 and directly output an output 406 indicative of one or more activities detected in the input image 402. The model 400 can be trained using a plurality of training images depicting the one or more activities. For example, the model 400 can be trained using a plurality of annotated training images. Each of the annotated images can depict a scene of an operating room and include one or more labels indicating one or more activities depicted in the scene. The plurality of annotated training images can comprise a video in which activities are bookmarked. At least some of the annotated images can be captured in the same operating room (e.g., operating room 100) for which the model will be deployed. During training, the model receives each image of the annotated images and provides an output indicative of detected activities. The output is compared against the labels associated with the image. Based on the comparison, the model 400 can be updated (e.g., via a backpropagation process).

With reference to FIG. 4B, a model 410 is configured to receive an input image 412 and output one or more detected objects and/or events 418 depicted in the input image 412. Based on the one or more detected objects and/or events 418, the system can determine, as output 416, one or more activities detected in the input image 412. The one or more machine learning models can be trained using a plurality of training images depicting the one or more objects and/or events. For example, the model 410 can be trained using a plurality of annotated training images. Each of the annotated images can depict a scene of an operating room and include one or more labels indicating objects and/or events depicted in the scene. At least some of the annotated images can be captured in the same operating room (e.g., operating room 100) for which the model will be deployed. During training, the model receives each image of the annotated images and provides an output indicative of one or more detected objects and/or events. The output is compared against the labels associated with the image. Based on the comparison, the model 400 can be updated (e.g., via a backpropagation process). The model 410 can be the same as model 310.

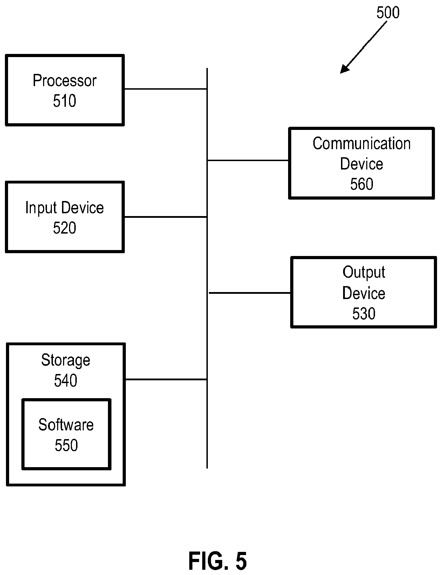

The operations described herein are optionally implemented by components depicted in FIG. 5. FIG. 5 illustrates an example of a computing device in accordance with one example. Device 500 can be a host computer connected to a network. Device 500 can be a client computer or a server. As shown in FIG. 5, device 500 can be any suitable type of microprocessor-based device, such as a personal computer, workstation, server or handheld computing device (portable electronic device) such as a phone or tablet. The device can include, for example, one or more of processor 510, input device 520, output device 530, storage 540, and communication device 560. Input device 520 and output device 530 can generally correspond to those described above, and can either be connectable or integrated with the computer. The system can include GPU in servers, client devices, edge computing devices, cloud computing devices, etc.

Input device 520 can be any suitable device that provides input, such as a touch screen, keyboard or keypad, mouse, or voice-recognition device. Output device 530 can be any suitable device that provides output, such as a touch screen, haptics device, or speaker.

Storage 540 can be any suitable device that provides storage, such as an electrical, magnetic or optical memory including a RAM, cache, hard drive, cloud storage, or removable storage disk. Communication device 560 can include any suitable device capable of transmitting and receiving signals over a network, such as a network interface chip or device. The components of the computer can be connected in any suitable manner, such as via a physical bus or wirelessly.

Software 550, which can be stored in storage 540 and executed by processor 510, can include, for example, the programming that embodies the functionality of the present disclosure (e.g., as embodied in the devices as described above).

Software 550 can also be stored and/or transported within any non-transitory computer-readable storage medium for use by or in connection with an instruction execution system, apparatus, or device, such as those described above, that can fetch instructions associated with the software from the instruction execution system, apparatus, or device and execute the instructions. In the context of this disclosure, a computer-readable storage medium can be any medium, such as storage 540, that can contain or store programming for use by or in connection with an instruction execution system, apparatus, or device.

Software 550 can also be propagated within any transport medium for use by or in connection with an instruction execution system, apparatus, or device, such as those described above, that can fetch instructions associated with the software from the instruction execution system, apparatus, or device and execute the instructions. In the context of this disclosure, a transport medium can be any medium that can communicate, propagate or transport programming for use by or in connection with an instruction execution system, apparatus, or device. The transport readable medium can include, but is not limited to, an electronic, magnetic, optical, electromagnetic or infrared wired or wireless propagation medium.

Device 500 may be connected to a network, which can be any suitable type of interconnected communication system. The network can implement any suitable communications protocol and can be secured by any suitable security protocol. The network can comprise network links of any suitable arrangement that can implement the transmission and reception of network signals, such as wireless network connections, T1 or T5 lines, cable networks, DSL, or telephone lines.

Device 500 can implement any operating system suitable for operating on the network. Software 550 can be written in any suitable programming language, such as C, C++, Java or Python. In various examples, application software embodying the functionality of the present disclosure can be deployed in different configurations, such as in a client/server arrangement, through an on-premise or cloud application, through a Web browser as a Web-based application or Web service, for example.

The disclosure will now be further described by the following numbered embodiments which are to be read in connection with the preceding paragraphs, and which do not limit the disclosure. The features, options and preferences as described above apply also to the following embodiments.

Embodiment 1. A method for determining non-compliance to surgical protocols in an operating room, the method comprising:
receiving one or more images of the operating room captured by one or more cameras;
detecting a surgical milestone associated with a surgery in the operating room using a first set of one or more trained machine-learning models based on the received one or more images;
detecting one or more activities in the operating room using a second set of one or more trained machine-learning models based on the received one or more images; and
determining, based on the detected one or more activities and a surgical protocol associated with the detected surgical milestone, that an instance of non-compliance to the surgical protocol has occurred in the operating room.

Embodiment 2. The method of Embodiment 1, further comprising: determining a severity level of the instance of non-compliance to the surgical protocol.

Embodiment 3. The method of Embodiment 2, further comprising:
determining that the severity level meets a predefined severity threshold;
in accordance with the determination that the determined severity level meets the predefined severity threshold: generating an alert.

Embodiment 4. The method of Embodiment 3, further comprising:
determining that the severity level does not meet the predefined severity threshold;
in accordance with the determination that the severity level does not meet the predefined severity threshold: foregoing generating the alert.

Embodiment 5. The method of Embodiment 3 or 4, wherein the alert is auditory, graphical, textual, haptic, or any combination thereof.

Embodiment 6. The method of any of Embodiments 1-5, further comprising: calculating an audit score for the surgery based on the instance of non-compliance to the surgical protocol.

Embodiment 7. The method of Embodiment 6, wherein the surgical milestone is a first surgical milestone of the surgery and the surgical protocol associated with the surgical milestone is a first surgical protocol, the method further comprising:
determining that an instance of non-compliance to a second surgical protocol associated with a second surgical milestone has occurred in the operating room; and
calculating the audit score for the surgery based on the instance of non-compliance to the first surgical protocol and the instance of non-compliance to the second surgical protocol.

Embodiment 8. The method of Embodiment 7, wherein the audit score is based on a weighted calculation of the instance of non-compliance to the first surgical protocol and the instance of non-compliance to the second surgical protocol.

Embodiment 9. The method of any of Embodiments 6-8, further comprising: comparing the audit score against a predefined audit score threshold.

Embodiment 10. The method of Embodiment 9, wherein the predefined audit score threshold is associated with a type of the surgery in the operating room.

Embodiment 11. The method of any of Embodiments 6-10, further comprising: displaying the audit score on a display; and saving the audit score to an electronic medical record.

Embodiment 12. The method of any of Embodiments 1-11, further comprising:
identifying a change to the surgical protocol; and
outputting a recommendation based on the identified change to the surgical protocol.
Embodiment 13. The method of Embodiment 12, wherein identifying a change to the surgical protocol comprises:
identifying a correlation between an outcome of the surgery in the operating room and the instance of non-compliance to the surgical protocol.

Embodiment 14. The method of any of Embodiments 1-13, further comprising: recommending retraining of the surgical protocol based on the instance of non-compliance to the surgical protocol.

Embodiment 15. The method of any of Embodiments 1-14, further comprising:
determining an identity or a surgical function of a person associated with the instance of non-compliance; and
determining whether to recommend a change to the surgical protocol or to recommend retraining of the surgical protocol at least partially based on the identity or the surgical function of the person associated with the instance of non-compliance.

Embodiment 16. The method of any of Embodiments 1-15, wherein the first set of one or more trained machine-learning models is the same as the second set of one or more trained machine-learning models.

Embodiment 17. The method of any of Embodiments 1-15, wherein the first set of one or more trained machine-learning models is different from the second set of one or more trained machine-learning models.

Embodiment 18. The method of any of Embodiments 1-17, wherein the one or more activities include:
linen changing on a surgical table;
cleaning of the surgical table;
wiping of the surgical table;
application of a disinfectant;
introduction of a surgical equipment;
preparation of the surgical equipment;
entrance of a person into the operating room;
exiting of the person out of the operating room;
opening of a door in the operating room;
closing of the door in the operating room;
donning of surgical attire;
contamination of sterile instruments;
contact between anything sterile and a non-sterile surface;
preparation of a patient;
usage of one or more blood units;
usage of one or more surgical sponges;
usage of one or more surgical swabs;
collection and/or disposal of waste;
fumigation;
sterile zone violation;
a conducted time-out;
a conducted debriefing;
fogging; or
any combination thereof.

Embodiment 19. The method of any of Embodiments 1-18, wherein the second set of one or more trained machine-learning models is configured to detect and/or track one or more objects in the operating room.

Embodiment 20. The method of Embodiment 19, wherein the one or more objects include:
one or more surgical tables;
one or more surgical lights;
one or more cleaning supplies;
one or more disinfectants;
one or more linens;
one or more surgical equipment;
one or more patients;
one or more medical staff members;
attire of the one or more medical staff members;
one or more doors in the operating room;
one or more blood units;
one or more surgical sponges;
one or more surgical swabs; or
any combination thereof.

Embodiment 21. The method of Embodiment 20, wherein the attire of the one or more medical staff members includes: a surgical mask, a surgical cap, a surgical glove, a surgical gown, or any combination thereof.

Embodiment 22. The method of Embodiment 20 or 21, wherein the one or more surgical equipment includes: one or more imaging devices, one or more monitoring devices, one or more surgical tools, or any combination thereof.

Embodiment 23. The method of any of Embodiments 1-22, further comprising: calculating a ratio between medical staff members and patients in the operating room.

Embodiment 24. The method of any of Embodiments 1-23, wherein detecting the surgical milestone comprises:
obtaining, from the first set of one or more trained machine-learning models, one or more detected objects or events; and
determining, based upon the one or more detected objects or events, the surgical milestone.

Embodiment 25. A system for determining non-compliance to surgical protocols in an operating room, the system comprising:
one or more processors;
a memory; and
one or more programs, wherein the one or more programs are stored in the memory and configured to be executed by the one or more processors, the one or more programs including instructions for:
   receiving one or more images of the operating room captured by one or more cameras;
   detecting a surgical milestone associated with a surgery in the operating room using a first set of one or more trained machine-learning models based on the received one or more images;
   detecting one or more activities in the operating room using a second set of one or more trained machine-learning models based on the received one or more images; and
   determining, based on the detected one or more activities and a surgical protocol associated with the detected surgical milestone, that an instance of non-compliance to the surgical protocol has occurred in the operating room.

Embodiment 26. The system of Embodiment 25, wherein the one or more programs further include instructions for: determining a severity level of the instance of non-compliance to the surgical protocol.

Embodiment 27. The system of Embodiment 26, wherein the one or more programs further include instructions for:
determining that the severity level meets a predefined severity threshold;
in accordance with the determination that the determined severity level meets the predefined severity threshold: generating an alert.

Embodiment 28. The system of Embodiment 27, wherein the one or more programs further include instructions for:
determining that the severity level does not meet the predefined severity threshold;
in accordance with the determination that the severity level does not meet the predefined severity threshold: foregoing generating the alert.

Embodiment 29. The system of Embodiment 27 or 28, wherein the alert is auditory, graphical, textual, haptic, or any combination thereof.

Embodiment 30. The system of any of Embodiments 25-29, wherein the one or more programs further include instructions for: calculating an audit score for the surgery based on the instance of non-compliance to the surgical protocol.

Embodiment 31. The system of Embodiment 30, wherein the surgical milestone is a first surgical milestone of the surgery and the surgical protocol associated with the surgical milestone is a first surgical protocol, and wherein the one or more programs further include instructions for:
determining that an instance of non-compliance to a second surgical protocol associated with a second surgical milestone has occurred in the operating room; and
calculating the audit score for the surgery based on the instance of non-compliance to the first surgical protocol and the instance of non-compliance to the second surgical protocol.

Embodiment 32. The system of Embodiment 31, wherein the audit score is based on a weighted calculation of the instance of non-compliance to the first surgical protocol and the instance of non-compliance to the second surgical protocol.

Embodiment 33. The system of any of Embodiments 30-32, wherein the one or more programs further include instructions for: comparing the audit score against a predefined audit score threshold.

Embodiment 34. The system of Embodiment 33, wherein the predefined audit score threshold is associated with a type of the surgery in the operating room.

Embodiment 35. The system of any of Embodiments 30-34, wherein the one or more programs further include instructions for:
displaying the audit score on a display; and
saving the audit score to an electronic medical record.

Embodiment 36. The system of any of Embodiments 25-35, wherein the one or more programs further include instructions for:
identifying a change to the surgical protocol; and
outputting a recommendation based on the identified change to the surgical protocol.

Embodiment 37. The system of Embodiment 36, wherein identifying a change to the surgical protocol comprises:
identifying a correlation between an outcome of the surgery in the operating room and the instance of non-compliance to the surgical protocol.

Embodiment 38. The system of any of Embodiments 25-37, wherein the one or more programs further include instructions for: recommending retraining of the surgical protocol based on the instance of non-compliance to the surgical protocol.

Embodiment 39. The system of any of Embodiments 25-38, wherein the one or more programs further include instructions for:
determining an identity or a surgical function of a person associated with the instance of non-compliance; and
determining whether to recommend a change to the surgical protocol or to recommend retraining of the surgical protocol at least partially based on the identity or the surgical function of the person associated with the instance of non-compliance.

Embodiment 40. The system of any of Embodiments 25-39, wherein the first set of one or more trained machine-learning models is the same as the second set of one or more trained machine-learning models.

Embodiment 41. The system of any of Embodiments 25-39, wherein the first set of one or more trained machine-learning models is different from the second set of one or more trained machine-learning models.

Embodiment 42. The system of any of Embodiments 25-41, wherein the one or more activities include:
linen changing on a surgical table;
cleaning of the surgical table;
wiping of the surgical table;
application of a disinfectant;
introduction of a surgical equipment;
preparation of the surgical equipment;
entrance of a person into the operating room;
exiting of the person out of the operating room;
opening of a door in the operating room;
closing of the door in the operating room;
donning of surgical attire;
contamination of sterile instruments;
contact between anything sterile and a non-sterile surface;
preparation of a patient;
usage of one or more blood units;
usage of one or more surgical sponges;
usage of one or more surgical swabs;
collection and/or disposal of waste;
fumigation;
sterile zone violation;
a conducted time-out;
a conducted debriefing;
fogging; or
any combination thereof.

Embodiment 43. The system of any of Embodiments 25-42, wherein the second set of one or more trained machine-learning models is configured to detect and/or track one or more objects in the operating room.

Embodiment 44. The system of Embodiment 43, wherein the one or more objects include:
one or more surgical tables;
one or more surgical lights;
one or more cleaning supplies;
one or more disinfectants;
one or more linens;
one or more surgical equipment;
one or more patients;
one or more medical staff members;
attire of the one or more medical staff members;
one or more doors in the operating room;
one or more blood units;
one or more surgical sponges;
one or more surgical swabs; or
any combination thereof.

Embodiment 45. The system of Embodiment 44, wherein the attire of the one or more medical staff members includes: a surgical mask, a surgical cap, a surgical glove, a surgical gown, or any combination thereof.

Embodiment 46. The system of Embodiment 44 or 45, wherein the one or more surgical equipment includes: one or more imaging devices, one or more monitoring devices, one or more surgical tools, or any combination thereof.

Embodiment 47. The system of any of Embodiments 25-46, wherein the one or more programs further include instructions for: calculating a ratio between medical staff members and patients in the operating room.

Embodiment 48. The system of any of Embodiments 25-47, wherein detecting the surgical milestone comprises:
obtaining, from the first set of one or more trained machine-learning models, one or more detected objects or events; and
determining, based upon the one or more detected objects or events, the surgical milestone.

Embodiment 49. A non-transitory computer-readable storage medium storing one or more programs, the one or more programs comprising instructions, which when executed by one or more processors of an electronic device, cause the electronic device to perform any of the methods of Embodiments 1-24.

Although the disclosure and examples have been fully described with reference to the accompanying figures, it is to be noted that various changes and modifications will become apparent to those skilled in the art. Such changes and modifications are to be understood as being included within the scope of the disclosure and examples as defined by the claims.

The foregoing description, for purpose of explanation, has been described with reference to specific examples. However, the illustrative discussions above are not intended to be exhaustive or to limit the invention to the precise forms disclosed. Many modifications and variations are possible in view of the above teachings. The examples were chosen and described in order to best explain the principles of the techniques and their practical applications. Others skilled in the art are thereby enabled to best utilize the techniques and various examples with various modifications as are suited to the particular use contemplated.

## Claims

1. A method for determining non-compliance to surgical protocols in an operating room, the method comprising:
receiving one or more images of the operating room captured by one or more cameras;
detecting a surgical milestone associated with a surgery in the operating room using a first set of one or more trained machine-learning models based on the received one or more images;
detecting one or more activities in the operating room using a second set of one or more trained machine-learning models based on the received one or more images; and
determining, based on the detected one or more activities and a surgical protocol associated with the detected surgical milestone, that an instance of non-compliance to the surgical protocol has occurred in the operating room.

2. The method of claim 1, further comprising: determining a severity level of the instance of non-compliance to the surgical protocol;
optionally comprising:
determining that the severity level meets a predefined severity threshold,
in accordance with the determination that the determined severity level meets the predefined severity threshold: generating an alert;
further optionally comprising:
determining that the severity level does not meet the predefined severity threshold,
in accordance with the determination that the severity level does not meet the predefined severity threshold: foregoing generating the alert; and/or
wherein optionally the alert is auditory, graphical, textual, haptic, or any combination thereof.

3. The method of claim 1 or 2, further comprising: calculating an audit score for the surgery based on the instance of non-compliance to the surgical protocol;
wherein optionally the surgical milestone is a first surgical milestone of the surgery and the surgical protocol associated with the surgical milestone is a first surgical protocol, the method further comprising:
determining that an instance of non-compliance to a second surgical protocol associated with a second surgical milestone has occurred in the operating room, and
calculating the audit score for the surgery based on the instance of non-compliance to the first surgical protocol and the instance of non-compliance to the second surgical protocol;
wherein further optionally the audit score is based on a weighted calculation of the instance of non-compliance to the first surgical protocol and the instance of non-compliance to the second surgical protocol.

4. The method of claim 3, further comprising:
a) comparing the audit score against a predefined audit score threshold,
wherein optionally the predefined audit score threshold is associated with a type of the surgery in the operating room; and/or
b) displaying the audit score on a display, and saving the audit score to an electronic medical record.

5. The method of any of claims 1-4, further comprising:
identifying a change to the surgical protocol; and
outputting a recommendation based on the identified change to the surgical protocol;
wherein optionally identifying a change to the surgical protocol comprises:
identifying a correlation between an outcome of the surgery in the operating room and the instance of non-compliance to the surgical protocol.

6. The method of any of claims 1-5, further comprising:
a) recommending retraining of the surgical protocol based on the instance of non-compliance to the surgical protocol; and/or
b) determining an identity or a surgical function of a person associated with the instance of non-compliance, and
determining whether to recommend a change to the surgical protocol or to recommend retraining of the surgical protocol at least partially based on the identity or the surgical function of the person associated with the instance of non-compliance.

7. The method of any of claims 1-6, wherein the first set of one or more trained machine-learning models is the same as, or different from, the second set of one or more trained machine-learning models.

8. The method of any of claims 1-7, wherein the one or more activities include:
linen changing on a surgical table;
cleaning of the surgical table;
wiping of the surgical table;
application of a disinfectant;
introduction of a surgical equipment;
preparation of the surgical equipment;
entrance of a person into the operating room;
exiting of the person out of the operating room;
opening of a door in the operating room;
closing of the door in the operating room;
donning of surgical attire;
contamination of sterile instruments;
contact between anything sterile and a non-sterile surface;
preparation of a patient;
usage of one or more blood units;
usage of one or more surgical sponges;
usage of one or more surgical swabs;
collection and/or disposal of waste;
fumigation;
sterile zone violation;
a conducted time-out;
a conducted debriefing;
fogging; or
any combination thereof.

9. The method of any of claims 1-8, wherein the second set of one or more trained machine-learning models is configured to detect and/or track one or more objects in the operating room;
wherein optionally the one or more objects include:
one or more surgical tables;
one or more surgical lights;
one or more cleaning supplies;
one or more disinfectants;
one or more linens;
one or more surgical equipment;
one or more patients;
one or more medical staff members;
attire of the one or more medical staff members;
one or more doors in the operating room;
one or more blood units;
one or more surgical sponges;
one or more surgical swabs; or
any combination thereof;
wherein further optionally the attire of the one or more medical staff members includes: a surgical mask, a surgical cap, a surgical glove, a surgical gown, or any combination thereof; and/or
wherein the one or more surgical equipment includes: one or more imaging devices, one or more monitoring devices, one or more surgical tools, or any combination thereof.

10. The method of any of claims 1-9, wherein detecting the surgical milestone comprises:
obtaining, from the first set of one or more trained machine-learning models, one or more detected objects or events; and
determining, based upon the one or more detected objects or events, the surgical milestone.

11. A system for determining non-compliance to surgical protocols in an operating room, the system comprising:
one or more processors;
a memory; and
one or more programs, wherein the one or more programs are stored in the memory and configured to be executed by the one or more processors, the one or more programs including instructions for:
receiving one or more images of the operating room captured by one or more cameras;
detecting a surgical milestone associated with a surgery in the operating room using a first set of one or more trained machine-learning models based on the received one or more images;
detecting one or more activities in the operating room using a second set of one or more trained machine-learning models based on the received one or more images; and
determining, based on the detected one or more activities and a surgical protocol associated with the detected surgical milestone, that an instance of non-compliance to the surgical protocol has occurred in the operating room.

12. The system of claim 11, wherein the one or more programs further include instructions for: determining a severity level of the instance of non-compliance to the surgical protocol;
wherein optionally the one or more programs further include instructions for:
determining that the severity level meets a predefined severity threshold;
in accordance with the determination that the determined severity level meets the predefined severity threshold: generating an alert;
wherein further optionally the one or more programs further include instructions for:
determining that the severity level does not meet the predefined severity threshold;
in accordance with the determination that the severity level does not meet the predefined severity threshold: foregoing generating the alert; and/or
wherein optionally the alert is auditory, graphical, textual, haptic, or any combination thereof.

13. The system of any of claims 11-12, wherein the one or more programs further include instructions for: calculating an audit score for the surgery based on the instance of non-compliance to the surgical protocol;
wherein optionally the surgical milestone is a first surgical milestone of the surgery and the surgical protocol associated with the surgical milestone is a first surgical protocol, and wherein the one or more programs further include instructions for:
determining that an instance of non-compliance to a second surgical protocol associated with a second surgical milestone has occurred in the operating room; and
calculating the audit score for the surgery based on the instance of non-compliance to the first surgical protocol and the instance of non-compliance to the second surgical protocol;
wherein further optionally the audit score is based on a weighted calculation of the instance of non-compliance to the first surgical protocol and the instance of non-compliance to the second surgical protocol.

14. The system of any of claims 11-13, wherein the one or more programs further include instructions for:
a) comparing the audit score against a predefined audit score threshold,
wherein optionally the predefined audit score threshold is associated with a type of the surgery in the operating room; and/or
b) displaying the audit score on a display, and
saving the audit score to an electronic medical record.

15. A computer program product comprising one or more programs, the one or more programs comprising instructions, which when executed by one or more processors of an electronic device, cause the electronic device to perform any of the methods of claims 1-10.
